# EUROPEAN PATENT APPLICATION

(11) **EP 3 708 677 A1**
(43) Date of publication of application: **16.09.2020**
(21) Application number: 20171554.7
(22) Date of filing: 18.07.2016
(51) Int. Cl.: C12Q 1/6818, C12Q 1/6848, C12Q 1/6806

(54) **METHODS AND COMPOSITIONS FOR CATALYTIC DNA ASSAYS**

(30) Priority: 17.07.2015 US 201562193649 P
(62) Divisional of application: 16828343.0
(71) Applicant: Luminex Corporation, Austin TX 78727 (US)
(72) Inventor: WHITMAN, Douglas, F., Round Rock, TX 78681 (US); WOHLERS, Travis, Austin, TX 78727 (US)
(74) Representative: D Young & Co LLP

(57) **Abstract**

Methods and compositions for assays using catalytic DNA (*e.g.,* that can cleave substrate nucleic acid sequences) are provided.

## Description

The present application claims the priority benefit of United States provisional application number 62/193,649, filed July 17, 2015, the entire contents of which is incorporated herein by reference.

### BACKGROUND OF THE INVENTION

### 1. Field of the Invention

The present invention relates generally to the field of molecular biology. More particularly, it concerns the detection and quantification of nucleic acids.

### 2. Description of Related Art

Polymerase chain reaction (PCR) is a molecular biology technique commonly used in medical and biological research labs for a variety of tasks, such as the detection of hereditary diseases, the identification of genetic fingerprints, the diagnosis of infectious diseases, the cloning of genes, paternity testing, and DNA computing. PCR has been accepted by molecular biologists as the method of choice for nucleic acid detection because of its unparalleled amplification and precision capability. DNA detection is typically performed at the end-point, or plateau phase of the PCR reaction, making it difficult to quantify the starting template. Real-time PCR or kinetic PCR advances the capability of end-point PCR analysis by recording the amplicon concentration as the reaction progresses. Amplicon concentration is most often recorded via a fluorescent signal change associated with the amplified target. Real-time PCR is also advantageous over end-point detection in that contamination is limited because it can be performed in a closed system. Other advantages include greater sensitivity, dynamic range, speed, and fewer processes required.

More recently, detection chemistries employing catalytic nucleic acids have been used in real-time PCR. Catalytic nucleic acids are capable of adopting structural configurations that confer enzymatic or catalytic activity that can cleave a substrate nucleic acid sequence. The ability to multiplex PCR using catalytic nucleic acids has, however, been limited to the number of fluorescent detection channels available in the thermocycler. For example, a multiplex reaction designed to detect 4 target sequences would require an instrument capable of distinguishing 4 different free floating fluorochromes by spectral differentiation, not including controls. These requirements not only limit the practical multiplexing capability, but also increase costs since such instruments typically require multiple lasers and filters. Various embodiments of the present invention overcome the limited multiplexing capability of assays that utilize catalytic nucleic acids.

### SUMMARY OF THE INVENTION

One embodiment provides a method comprising: (a) hybridizing a first component oligonucleotide and a second component oligonucleotide to a facilitator nucleic acid and a substrate nucleic acid to form a catalytically active nucleic acid enzyme; (b) cleaving the substrate nucleic acid with the catalytically active nucleic acid enzyme at a cleavage site to form a 5' fragment and a 3' fragment of the substrate nucleic acid; (c) hybridizing the 5' fragment of the substrate nucleic acid to a capture probe, wherein the capture probe comprises a first region that is complementary to the 5'-fragment of the substrate nucleic acid, a second region that provides a mismatch to the 3'-terminal nucleotide of the 5'-fragment of the substrate nucleic acid, and a third region that provides a template sequence for nucleic acid synthesis from the 3'-end of the 5'-fragment of the substrate nucleic acid; (d) removing the 3'-terminal nucleotide from the 5'-fragment of the substrate nucleic acid to form an extendable 5'-fragment of the substrate nucleic acid; and (e) extending the extendable 5'-fragment of the substrate nucleic acid along the capture probe. In some embodiments, the method further comprises performing melt analysis on a double-stranded extension product formed by extending the extendable 5'-fragment of the substrate nucleic acid along the capture probe.

One embodiment provides a method comprising: (a) hybridizing a first component oligonucleotide and a second component oligonucleotide to a facilitator nucleic acid and a substrate nucleic acid to form a catalytically active nucleic acid enzyme; (b) cleaving the substrate nucleic acid with the catalytically active nucleic acid enzyme at a cleavage site to form a 5' fragment and a 3' fragment of the substrate nucleic acid; (c) hybridizing the 5' fragment of the substrate nucleic acid to a capture probe, wherein the capture probe comprises a first region that is complementary to the 5'-fragment of the substrate nucleic acid and a second region that provides a template sequence for nucleic acid synthesis from the 3'-end of the 5'-fragment of the substrate nucleic acid; and (d) extending the 5'-fragment of the substrate nucleic acid along the capture probe. In embodiments where the cleavage of the substrate nucleic acid does not leave an extendable nucleotide at the 3' end, the method may further comprise removing the 3'-terminal nucleotide from the 5'-fragment of the substrate nucleic acid to form an extendable 5'-fragment of the substrate nucleic acid. In some embodiments, the method further comprises performing melt analysis on a double-stranded extension product formed by extending the 5'-fragment of the substrate nucleic acid along the capture probe.

Another embodiment provides a method comprising: (a) hybridizing at a first temperature a first component oligonucleotide and a second component oligonucleotide to a facilitator nucleic acid and a substrate nucleic acid to form a catalytically active nucleic acid enzyme, wherein the substrate nucleic acid comprises a tail at its 3' end that is not complementary to either of the first component oligonucleotide or the second component oligonucleotide; (b) cleaving the substrate nucleic acid with the catalytically active nucleic acid enzyme at a cleavage site to form a 5' fragment and a 3' fragment of the substrate nucleic acid; (c) hybridizing at a second temperature, which is lower than the first temperature, a primer to the tail of the 3' fragment of the substrate nucleic acid; and (d) extending the primer on the substrate nucleic acid, the 3'-fragment of the substrate nucleic acid, or both. In certain embodiments, the method further comprises performing melt analysis on a double-stranded extension product formed by extending the primer. The melt analysis may be used to distinguish cleaved substrate nucleic acids from uncleaved substrate nucleic acids, as well as to distinguigh among different substrate nucleic acids (whether cleaved or uncleaved). In some embodiments, one or both of the 3'-tail of the substrate nucleic acid and the primer are labeled. For example, the 3' tail of the substrate nucleic acid may be labeled with a fluorophore and the primer may be labeled with a quencher such that the signal from the fluorophore is quenched when the primer is hybridized to the 3' tail; or the 3' tail of the substrate nucleic acid may be labeled with a quencher and the primer may be labeled with a fluorophore.

One embodiment provides a composition comprising: (a) a facilitator nucleic acid; (b) a substrate nucleic acid; (c) a capture probe comprising a sequence partially complementary to the substrate nucleic acid; (d) a DNA polymerase having 3' exonuclease activity; (e) a first component oligonucleotide comprising a facilitator-specific sequence, a substrate-specific sequence, and a partial catalytic core sequence disposed between the target-specific sequence and the substrate-specific sequence; and (f) a second component oligonucleotide comprising a facilitator-specific sequence, a substrate-specific sequence, and a partial catalytic core sequence disposed between the target-specific sequence and the substrate-specific sequence; wherein the first component oligonucleotide and the second component form a catalytically active nucleic acid enzyme when hybridized to the facilitator nucleic acid and the substrate nucleic acid, and further wherein the catalytically active nucleic acid enzyme cleaves the substrate nucleic acid at a cleavage site to form a 5' fragment and a 3' fragment of the substrate nucleic acid. In some embodiments, the composition may further comprise a primer pair and a target nucleic acid, wherein the primer pair primes the synthesis of the facilitator nucleic acid from the target nucleic acid.

Another embodiment provides a composition comprising: (a) a facilitator nucleic acid; (b) a substrate nucleic acid comprising a first label; (c) a first primer complementary to a portion of the substrate nucleic acid and comprising a second label; (d) a first component oligonucleotide comprising a facilitator-specific sequence, a substrate-specific sequence, and a partial catalytic core sequence disposed between the target-specific sequence and the substrate-specific sequence; and (e) a second component oligonucleotide comprising a facilitator-specific sequence, a substrate-specific sequence, and a partial catalytic core sequence disposed between the target-specific sequence and the substrate-specific sequence; wherein the first component oligonucleotide and the second component form a catalytically active nucleic acid enzyme when hybridized to the facilitator nucleic acid and the substrate nucleic acid, and further wherein the catalytically active nucleic acid enzyme cleaves the substrate nucleic acid at a cleavage site to form a 5' fragment and a 3' fragment of the substrate nucleic acid. In some embodiments, the composition further comprises a target nucleic acid and a primer pair, the primer pair comprising a second primer and a third primer, wherein the primer pair primes the synthesis of the facilitator nucleic acid from the target nucleic acid. In certain embodiments, the Tms of both the second and third primers are above a predetermined Tm threshold, and the Tm of the first primer is below the predetermined Tm threshold.

In certain embodiments, the facilitator nucleic acid may be an amplicon. As such, the compositions disclosed herein may further comprise a primer or primers and the methods disclosed herein may further comprise performing PCR or another nucleic acid amplification technique prior to or concurrently with hybridizing the first component oligonucleotide and the second component oligonucleotide to the facilitator nucleic acid and the substrate nucleic acid to form the catalytically active nucleic acid enzyme, and cleaving the substrate nucleic acid with the catalytically active nucleic acid enzyme at the cleavage site to form the 5' fragment and the 3' fragment of the substrate nucleic acid.

In some embodiments where the composition comprises a primer(s) or the method comprises performing PCR or other amplification technique, at least one primer comprises: (a) a non-hybridizing region comprising natural nucleotides that are not complementary to the target nucleic acid; (b) an anchor region 5' of the non-hybridizing region, wherein the anchor region is complementary to a first portion of the target sequence; and (c) a footer region 3' of the non-hybridizing region, wherein the footer region is complementary to a second portion of the target sequence, and further wherein the Tm of the footer region and the second portion of the target nucleic acid is lower than the Tm of the anchor region and the first portion of the target sequence; and (d) a non-natural nucleotide region located adjacent the 5'-end of the footer region, within the footer region, or within the non-hybridizing region. In some embodiments, the primer comprises two non-natural nucleotide regions, wherein one of the non-natural nucleotide regions is located adjacent the 5'-end of the footer region or within the footer region, and the other of the non-natural nucleotide regions is located within the non-hybridizing region. In some embodiments, the non-natural nucleotide region consists of 1 or 2 non-natural nucleotides. In certain embodiments, the non-natural nucleotides are independently selected from the group consisting of isoC and isoG. In some embodiments, the facilitator-specific sequence of either the first or second component oligonucleotide comprises a sequence complementary to the non-hybridizing region of the primer. This may be used to increase the specificity of the component oligonucleotide for the facilitator nucleic acid created by amplification using primers with a non-hybridizing region. In embodiments where both primers in a primer pair comprise non-hybridizing regions, the facilitator-specific sequence of one of the first or second component oligonucleotide comprises a sequence complementary to the non-hybridizing region of one primer of the primer pair and the facilitator-specific sequence of the other component oligonucleotide comprises a sequence complementary to the non-hybridizing region of the other primer of the primer pair.

The methods disclosed herein may be performed as multiplex assays. For example, in one embodiment, the method comprises: (a) providing at least two different first component oligonucleotides, at least two different second component oligonucleotides, at least two different substrate nucleic acids, and at least two different capture probes, wherein the at least two different substrate nucleic acids are labeled with the same label but have nucleic acid sequences that differ from each other and are complementary to their respective complementary sequences in the at least two different capture probes, and wherein extension products formed by extending 3-ends of the 5'-fragments of the at least two different substrate nucleic acids on the at least two different capture probes can be distinguished by Tm; (b) if one or more facilitator nucleic acids complementary to one or more of the at least two different first and second component oligonucleotides is present in a sample, hybridizing the first component oligonucleotide and the second component oligonucleotide to the facilitator nucleic acid and the substrate nucleic acid to form the catalytically active nucleic acid enzyme; (c) cleaving the one or more substrate nucleic acids with the catalytically active nucleic acid enzyme at the cleavage site to form the 5' fragment and the 3' fragment of the substrate nucleic acid; (d) hybridizing the 5' fragment(s) of the one or more substrate nucleic acids to the one or more different capture probes; (e) if the 3'-terminal nucleotide is non-extendable (e.g., it lacks a 3' -OH), removing the 3'-terminal nucleotide from the 5'-fragment of the substrate nucleic acid to form an extendable 5'-fragment of the substrate nucleic acid; (f) extending the 5'-fragment of the substrate nucleic acid along the capture probe; and (g) performing melt analysis to identify the one or more facilitator nucleic acids present in the sample.

In another example of a multiplex embodiment, the method comprises: (a) providing at least two different first component oligonucleotides, at least two different second component oligonucleotides, at least two different substrate nucleic acids, and at least two different primers, wherein the at least two different substrate nucleic acids are labeled with the same label but have nucleic acid sequences that differ from each other and are complementary to their respective complementary sequences in the at least two different primers, and wherein extension products formed by extending the primers on the 3'-tails of the at least two different substrate nucleic acids can be distinguished by Tm; (b) if one or more facilitator nucleic acids complementary to one or more of the at least two different first and second component oligonucleotides is present in a sample, hybridizing the first component oligonucleotide and the second component oligonucleotide to the facilitator nucleic acid and the substrate nucleic acid to form the catalytically active nucleic acid enzyme; (c) cleaving the one or more substrate nucleic acids with the catalytically active nucleic acid enzyme at the cleavage site to form the 5' fragment and the 3' fragment of the substrate nucleic acid; (d) hybridizing the 3'-tail(s) of the one or more substrate nucleic acids to the one or more different primers; (e) extending the primer along the substrate nuclei acid, the 3'- fragment of the substrate nuclei acid, or both; and (f) performing melt analysis to identify the one or more facilitator nucleic acids present in the sample.

Compositions for performing such multiplex assays are also provided. For example, in one embodiment the composition comprises at least two different facilitator nucleic acids; at least two different substrate nucleic acids; at least two different capture probes, at least two different first component oligonucleotides, and at least two different second component oligonucleotides, wherein the at least two different substrate nucleic acids are labeled with the same label but have nucleic acid sequences that differ from each other and are complementary to their respective complementary sequences in the at least two different capture probes, and wherein the at least two different capture probes can be distinguished by Tm. In another embodiment, the composition comprises at least two different facilitator nucleic acids; at least two different tailed substrate nucleic acids; at least two different primers, at least two different first component oligonucleotides, and at least two different second component oligonucleotides, wherein the at least two different substrate nucleic acids are labeled with the same label but have nucleic acid sequences that differ from each other and are complementary to their respective complementary sequences in the at least two different primers, and wherein the extension products of the at least two different primers can be distinguished by Tm.

Such multiplex assays may be used to detect one or more of the 2, 3, 4, 5, 6, 7, 8, 9, 10, 12, 14, 16, 18, 20, 22, 24, 26, 28, 30, 32, 34, 36, 38, 40, 45, 50, 60, 70, 80, 90, 100, 150, 200, 250, 300, or any range derivable therein, target or facilitator nucleic acids potentially present in a sample. In particular embodiments, the identity of a particular target or facilitator nucleic acid is based on a combination of a signal from a label or combination of labels (e.g, fluorophores, quenchers, etc.) and a melt analysis.

A component oligonucleotide is a molecule that comprises a partial catalytic core sequence. A functional catalytic core can be reconstituted by bringing into proximity with each other the partial catalytic core sequences of two or more component oligonucleotides. In certain embodiments, the methods disclosed herein utilize a first component oligonucleotide that comprises a facilitator-specific sequence, a substrate-specific sequence, and a partial catalytic core sequence disposed between the target-specific sequence and the substrate-specific sequence. In certain embodiments, the methods disclosed herein utilize a second component oligonucleotide that comprises a facilitator-specific sequence, a substrate-specific sequence, and a partial catalytic core sequence disposed between the target-specific sequence and the substrate-specific sequence. The facilitator-specific sequences are designed to be complementary to a portion of the facilitator nucleic acid, and the length and composition of the facilitator-specific sequences can be adjusted to suit the desired hybridization conditions. In certain embodiments, a component oligonucleotide will have between about 6 to 30, 6 to 26, 6 to 24, 6 to 20, 6 to 12, 8 to 30, 8 to 24, 8 to 16, 10 to 30, 10 to 24, 10 to 20, or 10 to 15 nucleotides complementary to the facilitator nucleic acid.

The catalytic core sequence is divided between two or more component oligonucleotides such that each component oligonucleotide has only a partial catalytic core. The catalytic core can be reconstituted from the partial catalytic core sequences upon hybridization of the component oligonucleotides with the facilitator nucleic acid and substrate nucleic acid. In certain embodiments, the partial catalytic core sequence of a component oligonucleotide comprises between about 2 to 20, 2 to 16, 2 to 12, 4 to 12, 6 to 12, or 4 to 10 nucleotides.

The substrate nucleic acid comprises regions complementary to the substrate-specific sequence of each of the component oligonucleotides needed to reconstitute a catalytic core and a region that contains the cleavage site that is cleaved by the catalytic core. The substrate nucleic acid may comprise deoxyribonucleotides, ribonucleotides, non-natural nucleotides, or a combination thereof. In some embodiments, the cleavage site of the substrate nucleic acid comprises one or two ribonucleotides. In particular embodiments, the substrate nucleic acid comprises non-natural nucleotides selected from the group consisting of iso-guanine and iso-cytosine.

The substrate nucleic acid may also comprise one or more labels, which may be used to facilitate detection. For example, the substrate nucleic acid may comprise a fluorophore, a quencher, or both a fluorophore and a quencher, wherein the fluorophore and the quencher are disposed on different sides of the cleavage site.

In some embodiments, the substrate nucleic acid may further comprise a tail region at its 5' or 3' end. In certain embodiments, the tail region may be self-complementary, complementary to a capture probe, or complementary to a primer, and not complementary to the component oligonucleotides. In embodiments where the tail is complementary to the capture probe, the tail is preferably located at the 5' end of the substrate nucleic acid. In embodiments where the tail is complementary to the primer, the tail is preferably located at the 3' end of the substrate nucleic acid. The tail region may comprise one or more non-natural nucleotides. In embodiments where the substrate nucleic acid is labeled, the label may be located in the tail region. In some aspects, the tail region may be used to control the Tm of the substrate nucleic acid (or fragments thereof) with other molecules. For example, the tail may be located at the 5'-end of the substrate nucleic acid and the length and/or composition of the tail region may be configured such that the Tm of the substrate nucleic acid and the first and second component oligonucleotides is greater than the Tm of the 5' fragment of the substrate nucleic acid and the capture probe, which is greater than the Tm of the 5' fragment of the substrate nucleic acid and the first and second component oligonucleotides. Accordingly, an uncleaved substrate nucleic acid will more favorably hybridize to the component oligonucleotides, whereas the 5'-fragment of the cleaved substrate nucleic acid will more favorably hybridize to the capture probe than to a component oligonucleotide.

In some embodiments, capture probes have a first region that is complementary to a portion of the 5'-fragment of the substrate nucleic acid and a second region that provides a template sequence for nucleic acid synthesis from the 3'-end of the 5'-fragment of the substrate nucleic acid. In other embodiments, capture probes have a first region that is complementary to a portion of the 5'-fragment of the substrate nucleic acid, a second region the provides a mismatch to the 3'-terminal nucleotide of the 5'-fragment of the substrate nucleic acid, and a third region that provides a template sequence for nucleic acid synthesis from the 3'-end of the 5'-fragment of the substrate nucleic acid. The length of the first region that is complementary to the 5'-fragment of the substrate nucleic acid can be designed to accommodate the desired hybridization conditions. In some embodiments, the capture probe comprises a label such as a fluorophore or a quencher. The location of a fluorophore or quencher on the capture probe may be such that it interacts with a quencher or fluorophore on the 5'-fragment of the substrate nucleic acid when the two sequences are hybridized, thereby resulting in quenching of fluorescent signal when double-stranded and an unquenching of the signal when single-stranded.

Cleaving the substrate nucleic acid with the catalytically active nucleic acid enzyme at a cleavage site in many cases, produces a 5' fragment that lacks a 3' -OH group. As such, the 5' fragment cannot prime nucleic acid synthesis unless the 3'-terminal nucleotide is removed from the 5'-fragment of the substrate. In one embodiment, the 3'-terminal nucleotide is removed from the 5'-fragment of the substrate nucleic acid by a 3'-exonuclease activity (or proof reading activity) of a DNA polymerase. In this embodiment, the capture probe is designed to have a mismatch with the 3'-terminal nucleotide of the 5'-fragment, the proof reading activity of the polymerase will remove the mismatched nucleotide from the 5'-fragment, which will result in a new 3'-terminal nucleotide having a 3' -OH group that can prime nucleic acid synthesis. The 3'-terminal mismatch will typically be a mismatch of 1, 2, or 3 nucleotides. Longer mismatched regions at the 3'-end of the 5'-fragment may be used, but these would be more efficiently removed by another mechanism such as with a flap endonuclease. There are a number of commercially available DNA polymerases with 3'-exonuclease activity including, for example, One Taq®, Q5®, Phusion®, Vent®, Deep Vent®, and LongAmp®. In certain embodiments the DNA polymerase may also have 5'-exonuclease activity. In other embodiments, the DNA polymerase lacks 5'-exonuclease activity. In some embodiments, the DNA polymerase is a hotstart DNA polymerase.

Another embodiment provides primer comprising: (a) a non-hybridizing region comprising natural nucleotides that are not complementary to the target nucleic acid; (b) an anchor region 5' of the non-hybridizing region, wherein the anchor region is complementary to a first portion of the target sequence; (c) a footer region 3' of the non-hybridizing region, wherein the footer region is complementary to a second portion of the target sequence, and further wherein the Tm of the footer region and the second portion of the target nucleic acid is lower than the Tm of the anchor region and the first portion of the target sequence; and (d) a non-natural nucleotide region located adjacent the 5'-end of the footer region, within the footer region, or within the non-hybridizing region. In some embodiments, the non-natural nucleotide region is located one, two, or three nucleotides from the 3'-terminal nucleotide of the footer region.

In certain embodiments, the primer comprises two non-natural nucleotide regions, wherein one of the non-natural nucleotide regions is located adjacent the 5'-end of the footer region or within the footer region, and the other of the non-natural nucleotide regions is located within the non-hybridizing region. In certain aspects, the non-natural nucleotide region comprises 1 or more consecutive or non-consecutive non-natural nucleotides. In certain aspects, the non-natural nucleotide region consists of 1 or 2 non-natural nucleotides. In some embodiments, the non-natural nucleotides are independently selected from the group consisting of isoC and isoG.

The primers described above are useful for reducing primer-dimer formation, reducing off-target amplification, and accommodating polymorphic sequences at primer binding sites on target nucleic acid sequences, as well as skipping non-conserved regions within target genomes in a manner that does not favor priming of any specific sequence composition within the non-conserved region.

In one embodiment, there is provided a method for reducing primer-dimer formation during nucleic acid amplification comprising amplifying a target sequence in the presence of two or more primers wherein at least one of the two or more primers is a primer comprising: (a) a non-hybridizing region comprising natural nucleotides that are not complementary to the target nucleic acid; (b) an anchor region 5' of the non-hybridizing region, wherein the anchor region is complementary to a first portion of the target sequence; (c) a footer region 3' of the non-hybridizing region, wherein the footer region is complementary to a second portion of the target sequence, and further wherein the Tm of the footer region and the second portion of the target nucleic acid is lower than the Tm of the anchor region and the first portion of the target sequence; and (d) a non-natural nucleotide region located adjacent the 5'-end of the footer region, within the footer region, or within the non-hybridizing region.

In one embodiment, there is provided a method for reducing off-target amplification during nucleic acid amplification comprising amplifying a target sequence using at least one primer comprising: (a) a non-hybridizing region comprising natural nucleotides that are not complementary to the target nucleic acid; (b) an anchor region 5' of the non-hybridizing region, wherein the anchor region is complementary to a first portion of the target sequence; (c) a footer region 3' of the non-hybridizing region, wherein the footer region is complementary to a second portion of the target sequence, and further wherein the Tm of the footer region and the second portion of the target nucleic acid is lower than the Tm of the anchor region and the first portion of the target sequence; and (d) a non-natural nucleotide region located adjacent the 5'-end of the footer region, within the footer region, or within the non-hybridizing region.

In one embodiment, there is provided a method for accommodating polymorphic sequences at primer binding sites on target nucleic acid sequences comprising amplifying a target sequence using at least one primer comprising: (a) a non-hybridizing region comprising natural nucleotides that are not complementary to the target nucleic acid; (b) an anchor region 5' of the non-hybridizing region, wherein the anchor region is complementary to a first portion of the target sequence; (c) a footer region 3' of the non-hybridizing region, wherein the footer region is complementary to a second portion of the target sequence, and further wherein the Tm of the footer region and the second portion of the target nucleic acid is lower than the Tm of the anchor region and the first portion of the target sequence; and (d) a non-natural nucleotide region located adjacent the 5'-end of the footer region, within the footer region, or within the non-hybridizing region, wherein the non-hybridizing region of the primer spans the polymorphic sequences.

The target nucleic acid may be any sequence of interest. In some embodiments, the nucleic acid is a DNA. In some embodiments, the nucleic acid is an RNA. The sample containing the target nucleic acid may be any sample that contains nucleic acids. In certain aspects of the invention the sample is, for example, from a subject who is being screened for the presence or absence of one or more genetic mutations or polymorphisms. In another aspect of the invention the sample may be from a subject who is being tested for the presence or absence of a pathogen. Where the sample is obtained from a subject, it may be obtained by methods known to those in the art such as aspiration, biopsy, swabbing, venipuncture, spinal tap, fecal sample, or urine sample. In some aspects of the invention, the sample is an environmental sample such as a water, soil, or air sample. In other aspects of the invention, the sample is from a plant, bacteria, virus, fungi, protozoan, or metazoan. The term target nucleic acid encompasses both an unamplified sequence and amplicons thereof.

A primer is a nucleic acid that is capable of priming the synthesis of a nascent nucleic acid in a template-dependent process. A target-specific primer refers to a primer that has been designed to prime the synthesis of a particular target nucleic acid. A primer pair refers to two primers, commonly known as a forward primer and a reverse primer or as an upstream primer and a downstream primer, which are designed to amplify a target sequence between the binding sites of the two primers on a template nucleic acid molecule. In certain embodiments, the primer has a target-specific sequence that is between 10-40, 15-30, or 18-26 nucleotides in length. A probe is a nucleic acid that is capable of hybridizing to a complementary nucleic acid. A target-specific probe refers to a probe that has been designed to hybridize to a particular target nucleic acid. Probes present in the reaction may comprise a blocked 3' hydroxyl group to prevent extension of the probes by a polymerase. The 3' hydroxyl group may be blocked with, for example, a phosphate group, a 3' inverted dT, or a reporter. High stringency hybridization conditions may be selected that will only allow hybridization between sequences that are completely complementary.

Various aspects of the present invention employ a tail on the substrate nucleic acids, which is complementary to a sequence on a primer or a capture probe. In certain embodiments, the sequences of such tails and their complements may be selected from sets of complementary tag and anti-tag sequences. Which sequence in a complementary pair is called the "tag" and which is called the "anti-tag" is arbitrary. The tags and anti-tags are preferably non-cross hybridizing, *i.e.,* each tag and anti-tag should hybridize only to its complementary partner, and not to other tags or anti-tags in the same reaction. Preferably, the tags and anti-tags also will not hybridize to other nucleic acids in the sample during a reaction. The tag and anti-tag sequences are also preferably designed to be isothermic, *i.e.,* of similar optimal hybridization temperature, whereby all of the tag and anti-tag sequences in a multiplex reaction will have approximately the same Tm. The proper selection of non-cross hybridizing tag and anti-tag sequences is useful in assays, particularly assays in a highly parallel hybridization environment, that require stringent non-cross hybridizing behavior. In certain embodiments, the tag and anti-tag sequences are between 6 to 60, 8 to 50, 10 to 40, 10 to 20, 12 to 24, or 20 to 30 nucleotides in length. In some embodiments, the tag and anti-tag sequences are 12, 14, 16, or 24 nucleotides in length. A number of tag and tag complement (*i.e.,* anti-tag) sequences are known in the art and may be used in the present invention. For example, U.S. Patent 7,226,737, incorporated herein by reference, describes a set of 210 non-cross hybridizing tags and anti-tags. In addition, U.S. Patent 7,645,868, incorporated herein by reference, discloses a family of 1168 tag sequences with a demonstrated ability to correctly hybridize to their complementary sequences with minimal cross hybridization. A "universal" tag or anti-tag refers to a tag or anti-tag that has the same sequence across all reactions in a multiplex reaction.

Labels, which may also be referred to as reporters, are used in various embodiments disclosed herein. A label or reporter is a molecule that facilitates the detection of another molecule (*e.g.,* a nucleic acid) to which it is attached. Numerous reporter molecules that may be used to label nucleic acids are known. Direct reporter molecules include fluorophores, chromophores, and radiophores. Indirect reporter molecules include biotin, which must be bound to another molecule such as streptavidin-phycoerythrin for detection. Pairs of labels, such as fluorescence resonance energy transfer pairs or fluorophore-quencher pairs, may also be employed.

In some embodiments, non-natural bases that differ from the naturally occurring bases (A, T, C, G, and U) in their hydrogen bonding pattern may be incorporated into the primers, probes, component oligonucleotides, substrate nucleic acids, and facilitator nucleic acids described herein. One example is the isoC and isoG bases that hydrogen bond with each other, but not with natural bases. The incorporation of these non-natural bases in primers and/or probes is useful in reducing non-specific hybridization. Methods of using such non-natural bases to assay target nucleic acids are disclosed in U.S. Patent No. 6,977,161, which is incorporated herein by reference.

In certain aspects of the invention, a solid support is used. In particular, one ore more of the primers, probes, component oligonucleotides, substrate nucleic acids, and/or facilitator nucleic acids described herein may be attached to a solid support to facilitate detection and/or compartmentalization. A variety of solid supports for the immobilization of biomolecules are known. For example, the solid support may be nitrocellulose, nylon membrane, glass, activated quartz, activated glass, polyvinylidene difluoride (PVDF) membrane, polystyrene substrates, polyacrylamide-based substrate, other polymers, copolymers, or crosslinked polymers such as poly(vinyl chloride), poly(methyl methacrylate), poly(dimethyl siloxane), photopolymers (which contain photoreactive species such as nitrenes, carbenes and ketyl radicals capable of forming covalent links with target molecules). A solid support may be in the form of, for example, a bead (microsphere), a column, or a chip. Molecules immobilized on planar solid supports are typically identified by their spatial position on the support. Molecules immobilized on non-planar solid supports, such as particles or beads, are often identified by some form of encoding of the support, as discussed below. In some embodiments, a linker is placed between the target-specific probe or the anti-tag and the solid support to which it is attached.

Beads and particles may be encoded such that one subpopulation of beads or particles can be distinguished from another subpopulation. Encoding may be by a variety of techniques. For example, the beads may be fluorescently labeled with fluorescent dyes having different emission spectra and/or different signal intensities. In certain embodiments, the beads are Luminex MagPlex® microspheres or Luminex xMAP® microspheres. The size of the beads in a subpopulation may also be used to distinguish one subpopulation from another. Another method of modifying a bead is to incorporate a magnetically responsive substance, such as Fe₃O₄, into the structure. Paramagnetic and superparamagnetic microspheres have negligible magnetism in the absence of a magnetic field, but application of a magnetic field induces alignment of the magnetic domains in the microspheres, resulting in attraction of the microspheres to the field source. Combining fluorescent dyes, bead size, and/or magnetically responsive substances into the beads can further increase the number of different subpopulations of beads that can be created.

In certain aspects of the invention, an emulsion is used. In particular, digital PCR, droplet PCR, or emulsion PCR may be used in conjunction with the detection, signal generation, and multiplex signal generation components of the described embodiments. Emulsions may also be used with the described embodiments in the absence of PCR amplification.

In certain aspects of the invention amplification is performed by isothermal amplification. The cleavage of the substrate will cause the melting temperature of the cleaved strands to drop allowing them to melt from the component oligonucleotides. This can allow a new substrate to bind and cleave. These steps may be performed at a single reaction temperature. Other isothermal amplification methods may be used in addition to the isothermal signal generation capability of the described embodiments. These may include as non-limiting examples: Strand Displacement Amplification, Nicking Enzyme Amplification Reaction, Rolling Circle Amplification, Helicase Dependent Amplification, and the like. Other amplification methods such as those described in US20140017669, which is incorporated herein by reference, may also be used in emulsions which improve sensitivity by containing the signal to a localized area. The methods described in US20140017669, which is incorporated herein by reference, may also be used to produce sequences that may hybridize to a capture sequence, extend, and produce a specific melt signature indicative of the presence or absence of the target. They may also produce 5' and 3' cleavage products which may hybridize to another primer, and be extended to produce a specific melt signature indicative of the presence or absence of the target.

As used herein, "hybridization," "hybridizes" or "capable of hybridizing" is understood to mean the forming of a double or triple stranded molecule or a molecule with partial double or triple stranded nature. The term "anneal" as used herein is synonymous with "hybridize." As used herein "stringent conditions" or "high stringency" are those conditions that allow hybridization between or within one or more nucleic acid strands containing complementary sequences, but preclude hybridization of non-complementary sequences. Such conditions are well known to those of ordinary skill in the art, and are preferred for applications requiring high selectivity. Stringent conditions may comprise low salt and/or high temperature conditions. It is understood that the temperature and ionic strength of a desired stringency are determined in part by the length of the particular nucleic acids, the length and nucleobase content of the target sequences, the charge composition of the nucleic acids, and to the presence or concentration of formamide, tetramethylammonium chloride or other solvents in a hybridization mixture.

It is contemplated that any method or composition described herein can be implemented with respect to any other method or composition described herein.

The terms "comprise" (and any form of comprise, such as "comprises" and "comprising"), "have" (and any form of have, such as "has" and "having"), "contain" (and any form of contain, such as "contains" and "containing"), and "include" (and any form of include, such as "includes" and "including") are open-ended linking verbs. As a result, a method, composition, kit, or system that "comprises," "has," "contains," or "includes" one or more recited steps or elements possesses those recited steps or elements, but is not limited to possessing only those steps or elements; it may possess (*i.e.,* cover) elements or steps that are not recited. Likewise, an element of a method, composition, kit, or system that "comprises," "has," "contains," or "includes" one or more recited features possesses those features, but is not limited to possessing only those features; it may possess features that are not recited.

Any embodiment of any of the present methods, composition, kit, and systems may consist of or consist essentially of-rather than comprise/include/contain/have-the described steps and/or features. Thus, in any of the claims, the term "consisting of' or "consisting essentially of' may be substituted for any of the open-ended linking verbs recited above, in order to change the scope of a given claim from what it would otherwise be using the open-ended linking verb.

The use of the term "or" in the claims is used to mean "and/or" unless explicitly indicated to refer to alternatives only or the alternatives are mutually exclusive, although the disclosure supports a definition that refers to only alternatives and "and/or."

Throughout this application, the term "about" is used to indicate that a value includes the standard deviation of error for the device or method being employed to determine the value.

Following long-standing patent law, the words "a" and "an," when used in conjunction with the word "comprising" in the claims or specification, denotes one or more, unless specifically noted.

Other objects, features and advantages of the present invention will become apparent from the following detailed description. It should be understood, however, that the detailed description and the specific examples, while indicating specific embodiments of the invention, are given by way of illustration only, since various changes and modifications within the spirit and scope of the invention will become apparent to those skilled in the art from this detailed description.

### BRIEF DESCRIPTION OF THE DRAWINGS

The following drawings form part of the present specification and are included to further demonstrate certain aspects of the present invention. The invention may be better understood by reference to one or more of these drawings in combination with the detailed description of specific embodiments presented herein.
**FIG. 1** illustrates an embodiment of a multiplexed detection method.
**FIG. 2** illustrates an embodiment of a multiplexed detection method.

### DESCRIPTION OF ILLUSTRATIVE EMBODIMENTS

Various methods and composition disclosed herein make use of catalytic nucleic acids. A number of nucleic acid molecules capable of adopting structural configurations that confer enzymatic or catalytic activity have been discovered or developed through in vitro evolution technology. These catalytic nucleic acid molecules are often referred to as "DNAzymes," "ribozymes," or "MNAzymes." Catalytic nucleic acids, such as the hammerhead ribozyme, the 10:23 DNAzyme, and the 8:17 DNAzyme, have multiple domains - a conserved catalytic domain (catalytic core) flanked by two non-conserved substrate binding domains. The 10:23 and 8:17 DNAzymes are capable of cleaving nucleic acid substrates at specific RNA phosphodiester bonds (Santoro, S. and Joyce, G. (1997) A general purpose RNA cleaving DNA enzyme. Proc Natl Acad Sci USA. 94: 4262-4266). The 10:23 DNAzyme has a catalytic domain of 15 deoxynucleotides flanked by two substrate-recognition arms. The 8:17 DNAzyme has a catalytic domain of 14 deoxynucleotides flanked by two substrate-recognition arms.

A catalytic nucleic acid can cleave a nucleic acid substrate with a target sequence (i.e., a substrate nucleic acid) that meets minimum requirements. The substrate sequence must be substantially complementary to the substrate-recognition regions of the catalytic nucleic acid, and the substrate must contain a specific sequence at the site of cleavage. Specific sequence requirements at the cleavage site include, for example, a purine:pyrimidine ribonucleotide sequence for cleavage by the 10:23 DNAzyme (Santoro and Joyce, 1997), and the sequence uridine:X for the hammerhead ribozymes, wherein X can equal A, C, or U, but not G (Perriman, R., Delves, A. and Gerlach, W. L. (1992) Extended target-site specificity for a hammerhead ribozyme. Gene. 113(2): 157-63).

MNAzymes, which are described in US 8,394,946 (incorporated herein by reference), separate the catalytic core of catalytic nucleic acid molecules into two or more separate molecules. A notable result of this separation is that target recognition and substrate cleavage can now be dictated by two separate molecules - a facilitator molecule and a substrate molecule. MNAzymes have been employed in real-time PCR with some success (Mokany et al., (2013) MNAzyme qPCR with Superior Multiplexing Capacity. Molecular Diagnostics and Genetics. 59(2):419-426). The ability to multiplex PCR using MNAzymes has, however, been limited to the number of fluorescent detection channels available in the thermocycler. For example, Mokany *et al.* were limited to multiplex PCR assays of no more than five targets.

Certain embodiments of the present invention overcome the limited multiplexing capability of assays that utilize catalytic nucleic acids by employing melt analysis in the detection and/or identification of a nucleic acid of interest. Melt analysis assays utilize melt or anneal peaks to discriminate between different double-stranded nucleic acids. By creating sequences with unique melt profiles, multiplexing can be achieved in a single color channel, thus allowing even more multiplexing with multiple color channels. For example, by utilizing 5 distinguishable melt profiles with each of 6 spectrally distinct fluorophores, up to 30 different targets can be assayed in a single reaction.

The melting temperature may be determined by exposing the amplicon or nucleic acid complex to a gradient of temperatures and observing a signal from a label or labels. Optionally, the melting temperature may be determined by (a) reacting an amplicon with an intercalating agent at a gradient of temperatures and (b) observing a detectable signal from the intercalating agent. Distinguishable double-stranded nucleic acid molecules may have melting temperatures that differ by 1-10 °C, for example, at least about 1 °C, more preferably by at least about 2 °C, or even more preferably by at least about 4 °C from the melting temperature of any of the other double-stranded nucleic acid molecules.

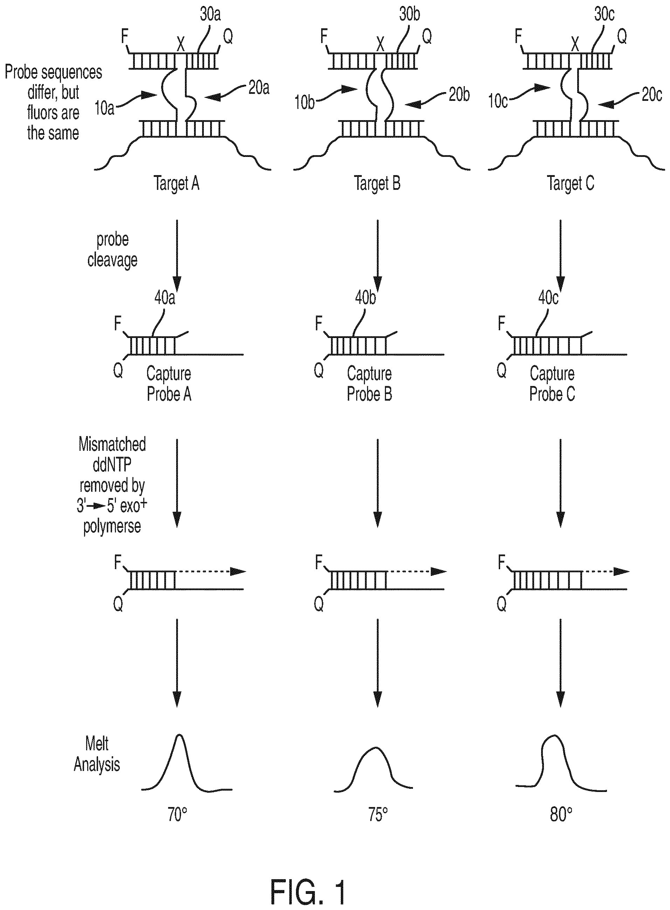

As illustrated in FIG. 1 for example, facilitator nucleic acids (in this case single-stranded amplicons labeled as Targets A, B, and C) each form a hybridization structure with three other nucleic acid molecules: a first component oligonucleotide (10a, 10b or 10c), a second component oligonucleotide (20a, 20b or 20c), and a substrate nucleic acid (30a, 30b, or 30c).

Each component oligonucleotide comprises a facilitator-specific sequence complementary to the facilitator nucleic acid, a substrate-specific sequence complementary to the substrate nucleic acid, and a partial catalytic core disposed between these two sequences. When the two partial catalytic sequences are brought together by hybridization to the facilitator nucleic acid and substrate nucleic acid, the substrate nucleic acid is cleaved (typically at a ribonucleotide, which is marked with an X in FIG. 1). In this embodiment, the substrate is labeled with a fluorophore (F) and a quencher (Q) on either side of the cleavage site (X). Thereby, the fluor is unquenched once the substrate is cleaved by the catalytic nucleic acids. In FIG. 1, substrates 30a, 30b, and 30c have different sequences but identical fluors. Although not shown in the figure, a short tail, which is complementary to the capture probe but not the component oligonucleotide, can be included at the 5' end of the substrate nucleic acid such that the fluorescently labeled 5' substrate fragment (40a, 40b or 40c) will preferentially hybridize to the capture probe. The uncleaved substrate nucleic acid should, however, have greater affinity for the component oligonucleotides than for the capture probe. In other words from highest Tm to lowest Tm, it would be: uncleaved substrate nucleic acid + both component oligonucleotides > 5' substrate fragment + capture probe > 5' substrate fragment + component oligonucleotide. Annealing of the component oligonucleotides fragment and capture probe for extension and melt analysis may optionally be performed at a lower temperature following PCR.

The fluorescently labeled 5' substrate fragment (40a, 40b or 40c) can then hybridize to a capture probe comprising a quencher. The length and composition of the capture probe is designed to produce a unique melt profile after the 5' substrate fragment has primed the extension of a complementary strand. However, the cleavage reaction discussed above leaves a 3'-nucleotide on the probe fragment that lacks a 3' -OH and, therefore, cannot be extended. Accordingly, the capture probe is designed with a mismatch at the position corresponding to the 3'-nucleotide of the probe fragment. In the presence of a polymerase having 3' exonuclease activity, this mismatch will be recognized and the ddNTP will be cleaved from the probe fragment. Extension can then proceed from the probe fragment. Finally, melt analysis identifies which target(s) are present in the reaction. In FIG. 1 Targets A, B, and C are shown to have melt peaks of 70° C, 75° C, and 80° C, respectively.

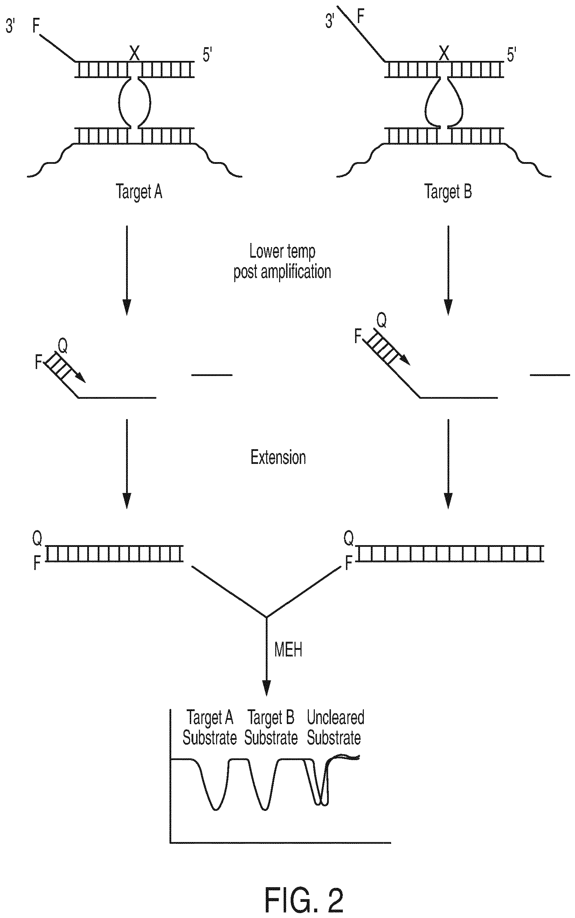

FIG. 2 illustrates an alternate embodiment in which the substrate nucleic acid comprises a region that hybridizes to the component oligonucleotides and a 3'overhang region that is complimentary to a low Tm primer. The 3' end of the overhang region contains one of either a fluorophore or quencher. The 5' end of the low Tm primer contains either a fluorophore or quencher to form a fluorophor/quencher pair with the 3' end of the reporter substrate. In FIG. 2 it is the 3' end of the overhang region that contains the fluorophore and the low Tm primer that contains the quencher. The Tm of the low Tm primer is below that of the lowest temperature in the amplification reaction that generates Target A and Target B, such that it does not hybridize and extend on the reporter substrate until after PCR amplification of Target A and Target B, at which point the temperature is lowered to a point that allows the low Tm primer to hybridize and extend on the substrate nucleic acid. Extension of the low Tm primer on the substrate will produce a double stranded product which is shorter if the substrate was cleaved, or longer if the substrate was not cleaved. This results in a specific melt signature for samples that contain a target that the component oligonucleotides reacted with. Multiple substrates with different Tms can be used in a single optical channel for the detection of more than one target. Tms of the double stranded products can be optimized by changing the composition of the 3' overhang portion of the substrate. The low Tm primer may be a universal primer for all overhang portions of all reporter substrates.

Typically, PCR applications employ a heat-stable DNA polymerase, such as Taq polymerase. This DNA polymerase enzymatically assembles a new DNA strand from nucleotides (dNTPs) using single-stranded DNA as template and DNA primers to initiate DNA synthesis. A basic PCR reaction requires several components and reagents including: a DNA template that contains the target sequence to be amplified; one or more primers, which are complementary to the DNA regions at the 5' and 3' ends of the target sequence; a DNA polymerase that preferably has a temperature optimum at around 70°C; deoxynucleotide triphosphates (dNTPs); a buffer solution providing a suitable chemical environment for optimum activity and stability of the DNA polymerase; divalent cations, typically magnesium ions (Mg²⁺); and monovalent cation potassium ions. As discussed above, where the 5'-fragment of the substrate nucleic acid is being used as a primer a polymerase with 3' exonuclease activity is used to remove the 3'-terminal ddNTP.

The majority of PCR methods use thermal cycling to subject the PCR sample to a defined series of temperature steps. Each cycle typically has 2 or 3 discrete temperature steps. The cycling is often preceded by a single temperature step ("initiation") at a high temperature (>90°C), and followed by one or two temperature steps at the end for final product extension ("final extension") or brief storage ("final hold"). The temperatures used and the length of time they are applied in each cycle depend on a variety of parameters. These include the enzyme used for DNA synthesis, the concentration of divalent ions and dNTPs in the reaction, and the melting temperature (Tm) of the primers. Commonly used temperatures for the various steps in PCR methods are: initialization step - 94-96°C; denaturation step - 94-98°C; annealing step - 50-65°C; extension/elongation step - 70-74°C; final elongation - 70-74°C; final hold - 4-10°C.

Real-time polymerase chain reaction, also called quantitative real time polymerase chain reaction (qPCR) or kinetic polymerase chain reaction, is used to amplify and simultaneously quantify a targeted DNA molecule. It enables both detection and quantification (as absolute number of copies or relative amount when normalized to DNA input or additional normalizing genes) of a specific sequence in a DNA sample. Real-time PCR may be combined with reverse transcription polymerase chain reaction to quantify low abundance RNAs. Relative concentrations of DNA present during the exponential phase of real-time PCR are determined by plotting fluorescence against cycle number on a logarithmic scale. Amounts of DNA may then be determined by comparing the results to a standard curve produced by real-time PCR of serial dilutions of a known amount of DNA.

Digital PCR (dPCR) involves partitioning the sample such that individual nucleic acid molecules contained in the sample are localized in many separate regions, such as in individual wells in microwell plates, in the dispersed phase of an emulsion, or arrays of nucleic acid binding surfaces. Each partition will contain 0 or 1 molecule, providing a negative or positive reaction, respectively. Unlike conventional PCR, dPCR is not dependent on the number of amplification cycles to determine the initial amount of the target nucleic acid in the sample. Accordingly, dPCR eliminates the reliance on exponential data to quantify target nucleic acids and provides absolute quantification.

Multiplex-PCR and multiplex real-time PCR use of multiple, unique primer sets within a single PCR reaction to produce amplicons of different DNA sequences. By targeting multiple genes at once, additional information may be gained from a single test run that otherwise would require several times the reagents and more time to perform. Annealing temperatures for each of the primer sets should be optimized to work within a single reaction.

Certain embodiments of the present invention also provide primers that (1) make the primers less susceptible to primer-dimer or other off-target amplifications, (2) increase amplicon specificity to the facilitator-specific sequences of the component oligonucleotides discussed above, and (3) can address polymorphic regions in some target sequences. The primers comprise an anchor region at the 5' end of the primer with a Tm capable of hybridizing to a first portion of a target sequence at a given annealing temperature; a non-hybridizing region comprising natural nucleotides, wherein the non-hybridizing region is not complimentary to the target sequence; a footer region at the 3-end of the primer that is capable of hybridizing to a second portion of the target sequence at a given annealing temperature, wherein the Tm of the footer region and the second portion of the target sequence is lower than the Tm of the anchor region and the first portion of the target sequence; and a non-natural nucleotide region. The non-natural nucleotide region may be located adjacent to the 5'-end of the footer region, within the footer region, or within the non-hybridizing region in order to prevent primer dimer formation. In some embodiments there may be two non-natural nucleotide regions, one in or adjacent the footer and the other in the non-hybridizing region. The non-natural nucleotide region will typically comprise 1 or 2 no-natural nucleotides, which may be for example, an iso-C or iso-G. The facilitator-specific sequences of the component oligonucleotides may be designed to be complementary to amplicon sequences that are complementary to the non-hybridizing region and non-natural nucleotide region of the primer, thereby increasing the specificity of the component oligonucleotides for the facilitator nucleic acids.

All of the compositions and methods disclosed and claimed herein can be made and executed without undue experimentation in light of the present disclosure. While the compositions and methods of this invention have been described in terms of certain embodiments, it will be apparent to those of skill in the art that variations may be applied to the compositions and methods and in the steps or in the sequence of steps of the methods described herein without departing from the concept, spirit and scope of the invention. More specifically, it will be apparent that certain agents which are both chemically and physiologically related may be substituted for the agents described herein while the same or similar results would be achieved. All such similar substitutes and modifications apparent to those skilled in the art are deemed to be within the spirit, scope and concept of the invention as defined by the appended claims.

Various preferred features and embodiments of the present invention will now be described with reference to the following numbered paragraphs (paras).
1. A method comprising:
   (a) hybridizing a first component oligonucleotide and a second component oligonucleotide to a facilitator nucleic acid and a substrate nucleic acid to form a catalytically active nucleic acid enzyme;
   (b) cleaving the substrate nucleic acid with the catalytically active nucleic acid enzyme at a cleavage site to form a 5' fragment and a 3' fragment of the substrate nucleic acid;
   (c) hybridizing the 5' fragment of the substrate nucleic acid to a capture probe, wherein the capture probe comprises a first region that is complementary to the 5'-fragment of the substrate nucleic acid, a second region that provides a mismatch to the 3'-terminal nucleotide of the 5'-fragment of the substrate nucleic acid, and a third region that provides a template sequence for nucleic acid synthesis from the 3'-end of the 5'-fragment of the substrate nucleic acid;
   (d) removing the 3'-terminal nucleotide from the 5'-fragment of the substrate nucleic acid to form an extendable 5'-fragment of the substrate nucleic acid; and
   (e) extending the extendable 5'-fragment of the substrate nucleic acid along the capture probe.
2. The method of para 1, wherein first component oligonucleotide comprises a facilitator-specific sequence, a substrate-specific sequence, and a partial catalytic core sequence disposed between the target-specific sequence and the substrate-specific sequence.
3. The method of para 1, wherein the second component oligonucleotide comprising a facilitator-specific sequence, a substrate-specific sequence, and a partial catalytic core sequence disposed between the target-specific sequence and the substrate-specific sequence.
4. The method of para 1, wherein the substrate nucleic acid comprises deoxyribonucleotides and ribonucleotides.
5. The method of para 1, wherein the substrate nucleic acid comprises one or more non-natural nucleotides.
6. The method of para 1, wherein the substrate nucleic acid comprises a fluorophore and a quencher, wherein the fluorophore and the quencher are disposed on different sides of the cleavage site.
7. The method of para 1, wherein the substrate nucleic acid further comprises a tail region at its 5' end, wherein the tail region is complementary to the capture probe and is not complementary to the first or second component oligonucleotides.
8. The method of para 7, wherein the tail region comprises one or more non-natural nucleotides.
9. The method of para 7, wherein the Tm of the substrate nucleic acid and the first and second component oligonucleotides is greater than the Tm of the 5' fragment of the substrate nucleic acid and the capture probe, which is greater than the Tm of the 5' fragment of the substrate nucleic acid and the first and second component oligonucleotides.
10. The method of para 1, wherein the cleavage site of the substrate nucleic acid comprises one or two ribonucleotides.
11. The method of para 1, wherein the facilitator-specific sequence of the first component oligonucleotide comprises between about 10 to 20 nucleotides or 6 to 24 nucleotides complementary to the facilitator nucleic acid.
12. The method of para 1, wherein the facilitator-specific sequence of the second component oligonucleotide comprises between about 10 to 20 nucleotides or 6 to 24 nucleotides complementary to the facilitator nucleic acid.
13. The method of para 1, wherein the partial catalytic core sequence of the first component oligonucleotide comprises between about 2 to 12 nucleotides.
14. The method of para 1, wherein the partial catalytic core sequence of the second component oligonucleotide comprises between about 2 to 12 nucleotides.
15. The method of any of paras 1 to 14, wherein the nucleotides are deoxyribonucleotides, ribonucleotides, non-natural nucleotides, or any combination thereof.
16. The method of para 1, wherein the capture probe comprises a fluorophore or a quencher.
17. The method of para 1, wherein the 3'-terminal nucleotide is removed from the 5'-fragment of the substrate nucleic acid by a 3'-exonuclease activity of a DNA polymerase.
18. The method of para 1, further comprising performing melt analysis on a double-stranded extension product formed by extending the extendable 5'-fragment of the substrate nucleic acid along the capture probe.
19. The method of any of paras 1 to 18, wherein the method comprises:
   (a) providing at least two different first component oligonucleotides, at least two different second component oligonucleotides, at least two different substrate nucleic acids, and at least two different capture probes, wherein the at least two different substrate nucleic acids are labeled with the same label but have nucleic acid sequences that differ from each other and are complementary to their respective complementary sequences in the at least two different capture probes, and wherein extension products formed by extending 5'-fragments of the at least two different substrate nucleic acids on the at least two different capture probes can be distinguished by Tm;
   (b) if one or more facilitator nucleic acids complementary to one or more of the at least two different first and second component oligonucleotides is present in a sample, hybridizing the first component oligonucleotide and the second component oligonucleotide to the facilitator nucleic acid and the substrate nucleic acid to form the catalytically active nucleic acid enzyme;
   (c) cleaving the one or more substrate nucleic acids with the catalytically active nucleic acid enzyme at the cleavage site to form the 5' fragment and the 3' fragment of the substrate nucleic acid;
   (d) hybridizing the 5' fragment(s) of the one or more substrate nucleic acids to the one or more different capture probes;
   (e) removing the 3'-terminal nucleotide from the 5'-fragment of the substrate nucleic acid to form an extendable 5'-fragment of the substrate nucleic acid;
   (f) extending the extendable 5'-fragment of the substrate nucleic acid along the capture probe; and
   (g) performing melt analysis to identify the one or more facilitator nucleic acids present in the sample.
20. The method of any of paras 1 to 19, wherein the facilitator nucleic acid is an amplicon and the method further comprises performing PCR prior to or concurrently with hybridizing the first component oligonucleotide and the second component oligonucleotide to the facilitator nucleic acid and the substrate nucleic acid to form the catalytically active nucleic acid enzyme, and cleaving the substrate nucleic acid with the catalytically active nucleic acid enzyme at the cleavage site to form the 5' fragment and the 3' fragment of the substrate nucleic acid.
21. A method comprising:
   (a) hybridizing at a first temperature a first component oligonucleotide and a second component oligonucleotide to a facilitator nucleic acid and a substrate nucleic acid to form a catalytically active nucleic acid enzyme, wherein the substrate nucleic acid comprises a tail at its 3' end that is not complementary to either of the first component oligonucleotide or the second component oligonucleotide;
   (b) cleaving the substrate nucleic acid with the catalytically active nucleic acid enzyme at a cleavage site to form a 5' fragment and a 3' fragment of the substrate nucleic acid;
   (c) hybridizing at a second temperature, which is lower than the first temperature, a primer to the 3' tail of the substrate nucleic acid; and
   (d) extending the primer on the substrate nucleic acid or fragment thereof.
22. The method of para 21, further comprising performing melt analysis on a double-stranded extension product formed by extending the primer.
23. The method of para 21 or 22, wherein the 3' tail of the substrate nucleic acid is labeled with a fluorophore and the 5' end of the primer is labeled with a quencher.
24. The method of para 20 or 21, wherein the 3' tail of the substrate nucleic acid is labeled with a quencher and the 5'end of the primer is labeled with a fluorophore.
25. The method of any of paras 21 to 24, wherein the method comprises:
   (a) providing at least two different first component oligonucleotides, at least two different second component oligonucleotides, at least two different substrate nucleic acids, and at least two different primers, wherein the at least two different substrate nucleic acids are labeled with the same label but have nucleic acid sequences that differ from each other and are complementary to their respective complementary sequences in the at least two different primers, and wherein extension products formed by extending the primers on the at least two different substrate nucleic acids can be distinguished by Tm;
   (b) if one or more facilitator nucleic acids complementary to one or more of the at least two different first and second component oligonucleotides is present in a sample, hybridizing the first component oligonucleotide and the second component oligonucleotide to the facilitator nucleic acid and the substrate nucleic acid to form the catalytically active nucleic acid enzyme;
   (c) cleaving the one or more substrate nucleic acids with the catalytically active nucleic acid enzyme at the cleavage site to form the 5' fragment and the 3' fragment of the substrate nucleic acid;
   (d) hybridizing the 3' tails of the one or more substrate nucleic acids to their respective primers;
   (e) extending the primers along the substrate nucleic acids, the 3'-fragments of the substrate nucleic acids, or both; and
   (f) performing melt analysis wherein the presence of the facilitator is detectable by identifying the melt signature associated with a primer that has extended along a cleaved substrate.
26. A composition comprising:
   (a) a facilitator nucleic acid;
   (b) a substrate nucleic acid;
   (c) a capture probe comprising a sequence partially complementary to the substrate nucleic acid;
   (d) a DNA polymerase having 3' exonuclease activity;
   (e) a first component oligonucleotide comprising a facilitator-specific sequence, a substrate-specific sequence, and a partial catalytic core sequence disposed between the target-specific sequence and the substrate-specific sequence; and
   (f) a second component oligonucleotide comprising a facilitator-specific sequence, a substrate-specific sequence, and a partial catalytic core sequence disposed between the target-specific sequence and the substrate-specific sequence;
   wherein the first component oligonucleotide and the second component form a catalytically active nucleic acid enzyme when hybridized to the facilitator nucleic acid and the substrate nucleic acid, and further wherein the catalytically active nucleic acid enzyme cleaves the substrate nucleic acid at a cleavage site to form a 5' fragment and a 3' fragment of the substrate nucleic acid.
27. A composition comprising:
   (a) a facilitator nucleic acid;
   (b) a substrate nucleic acid comprising a first label;
   (c) a first primer complementary to a portion of the substrate nucleic acid and comprising a second label;
   (d) a first component oligonucleotide comprising a facilitator-specific sequence, a substrate-specific sequence, and a partial catalytic core sequence disposed between the target-specific sequence and the substrate-specific sequence; and
   (e) a second component oligonucleotide comprising a facilitator-specific sequence, a substrate-specific sequence, and a partial catalytic core sequence disposed between the target-specific sequence and the substrate-specific sequence;
   wherein the first component oligonucleotide and the second component form a catalytically active nucleic acid enzyme when hybridized to the facilitator nucleic acid and the substrate nucleic acid, and further wherein the catalytically active nucleic acid enzyme cleaves the substrate nucleic acid at a cleavage site to form a 5' fragment and a 3' fragment of the substrate nucleic acid.
28. The composition of para 26 wherein the facilitator nucleic acid is an amplicon.
29. The composition of para 28, further comprising a primer pair and a target nucleic acid, wherein the primer pair primes the synthesis of the amplicon from the target nucleic acid.
30. The composition of para 27, wherein the facilitator nucleic acid is an amplicon.
31. The composition of para 30, further comprising target nucleic acid and a primer pair, the primer pair comprising a second primer and a third primer, wherein the primer pair primes the synthesis of the amplicon from the target nucleic acid.
32. The composition of para 31,wherein the Tms of both the second and third primers are above a predetermined Tm threshold, and the Tm of the first primer is below the predetermined Tm threshold.
33. The composition of para 26 or 27, wherein the substrate nucleic acid comprises deoxyribonucleotides and ribonucleotides.
34. The composition of para 26 or 27, wherein the substrate nucleic acid comprises one or more non-natural nucleotides.
35. The composition of para 26 or 27, wherein the substrate nucleic acid comprises a fluorophore and a quencher, wherein the fluorophore and the quencher are disposed on different sides of the cleavage site.
36. The composition of para 26, wherein the substrate nucleic acid further comprises a tail region at its 5' end, wherein the tail region is complementary to the capture probe and is not complementary to the first or second component oligonucleotides.
37. The composition of para 36, wherein the tail region comprises one or more non-natural nucleotides.
38. The composition of para 36, wherein the Tm of the substrate nucleic acid and the first and second component oligonucleotides is greater than the Tm of the 5' fragment of the substrate nucleic acid and the capture probe, which is greater than the Tm of the 5' fragment of the substrate nucleic acid and the first and second component oligonucleotides.
39. The composition of para 26 or 27, wherein the cleavage site of the substrate nucleic acid comprises one or two ribonucleotides.
40. The composition of para 26 or 27, wherein the facilitator-specific sequence of the first component oligonucleotide comprises between about 10 to 20 nucleotides complementary to the facilitator nucleic acid.
41. The composition of para 26 or 27, wherein the facilitator-specific sequence of the second component oligonucleotide comprises between about 10 to 20 nucleotides complementary to the facilitator nucleic acid.
42. The composition of para 26 or 27, wherein the substrate-specific sequence of the first component oligonucleotide comprises between about 6 to 24 nucleotides complementary to the substrate nucleic acid.
43. The composition of para 26 or 27, wherein the substrate-specific sequence of the second component oligonucleotide comprises between about 6 to 24 nucleotides complementary to the substrate nucleic acid.
44. The composition of para 26 or 27, wherein the partial catalytic core sequence of the first component oligonucleotide comprises between about 2 to 12 nucleotides.
45. The composition of para 26 or 27, wherein the partial catalytic core sequence of the second component oligonucleotide comprises between about 2 to 12 nucleotides.
46. The composition of any of paras 40 to 45, wherein the nucleotides are deoxyribonucleotides, ribonucleotides, non-natural nucleotides, or any combination thereof.
47. The composition of para 26, wherein the capture probe comprises a first region that is complementary to the 5'-fragment of the substrate nucleic acid, a second region the provides a mismatch to the 3'-terminal nucleotide of the 5'-fragment of the substrate nucleic acid, and a third region that provides a template sequence for nucleic acid synthesis from the 3'-end of the 5'-fragment of the substrate nucleic acid.
48. The composition of para 26, wherein the capture probe comprises a fluorophore or a quencher.
49. The composition of para 26, wherein the composition comprises at least two different facilitator nucleic acids; at least two different substrate nucleic acids; at least two different capture probes, at least two different first component oligonucleotides, and at least two different second component oligonucleotides, wherein the at least two different substrate nucleic acids are labeled with the same label but have nucleic acid sequences that differ from each other and are complementary to their respective complementary sequences in the at least two different capture probes, and wherein the at least two different capture probes can be distinguished by Tm.
50. The composition of para 29 or 31, wherein at least one primer of the primer pair comprises:
   (a) a non-hybridizing region comprising natural nucleotides that are not complementary to the target nucleic acid;
   (b) an anchor region 5' of the non-hybridizing region, wherein the anchor region is complementary to a first portion of the target sequence; and
   (c) a footer region 3' of the non-hybridizing region, wherein the footer region is complementary to a second portion of the target sequence, and further wherein the Tm of the footer region and the second portion of the target nucleic acid is lower than the Tm of the anchor region and the first portion of the target sequence; and
   (d) a non-natural nucleotide region located adjacent the 5'-end of the footer region, within the footer region, or within the non-hybridizing region.
51. The composition of para 50, wherein the primer comprises two non-natural nucleotide regions, wherein one of the non-natural nucleotide regions is located adjacent the 5'-end of the footer region or within the footer region, and the other of the non-natural nucleotide regions is located within the non-hybridizing region.
52. The composition of para 50, wherein the non-natural nucleotide region consists of 1 or 2 non-natural nucleotides.
53. The composition of para 52, wherein the non-natural nucleotides are independently selected from the group consisting of isoC and isoG.
54. The composition of para 50, wherein the target-specific sequence of either the first or second component oligonucleotide comprises a sequence complementary to the non-hybridizing region of the primer.
55. The composition of para 27, wherein the first label is a fluorophore and the second label is a quencher.
56. The composition of para 27, wherein the first label is a quencher and the second label is a fluorophore.
57. A primer comprising:
   (a) a non-hybridizing region comprising natural nucleotides that are not complementary to the target nucleic acid;
   (b) an anchor region 5' of the non-hybridizing region, wherein the anchor region is complementary to a first portion of the target sequence; and
   (c) a footer region 3' of the non-hybridizing region, wherein the footer region is complementary to a second portion of the target sequence, and further wherein the Tm of the footer region and the second portion of the target nucleic acid is lower than the Tm of the anchor region and the first portion of the target sequence; and
   (d) a non-natural nucleotide region located adjacent the 5'-end of the footer region, within the footer region, or within the non-hybridizing region.
58. The primer of para 57, wherein the non-natural nucleotide region is located adjacent the 5'-end of the footer region.
59. The primer of para 57, wherein the non-natural nucleotide region is located within the footer region.
60. The primer of para 59, wherein the non-natural nucleotide region is located one, two, or three nucleotides from the 3'-terminal nucleotide of the footer region.
61. The primer of para 57, wherein the non-natural nucleotide region is located within the non-hybridizing region.
62. The primer of para 57, wherein the primer comprises two non-natural nucleotide regions, wherein one of the non-natural nucleotide regions is located adjacent the 5'-end of the footer region or within the footer region, and the other of the non-natural nucleotide regions is located within the non-hybridizing region.
63. The composition of para 57, wherein the non-natural nucleotide region consists of 1 or 2 non-natural nucleotides.
64. The composition of para 57, wherein the non-natural nucleotides are independently selected from the group consisting of isoC and isoG.
65. A method for reducing primer-dimer formation during nucleic acid amplification comprising amplifying a target sequence in the presence of two or more primers wherein at least one of the two or more primers is a primer according to any of paras 57 to 64.
66. A method for reducing off-target amplification during nucleic acid amplification comprising amplifying a target sequence using at least one primer according to any of paras 57 to 64.
67. A method for accommodating polymorphic sequences at primer binding sites on target nucleic acid sequences comprising amplifying a target sequence using at least one primer according to any of paras 57 to 64, wherein the non-hybridizing region of the primer spans the polymorphic sequences.

## Claims

1. A composition comprising:
(a) a facilitator nucleic acid;
(b) a substrate nucleic acid;
(c) a capture probe comprising a sequence partially complementary to the substrate nucleic acid;
(d) a DNA polymerase having 3' exonuclease activity;
(e) a first component oligonucleotide comprising a facilitator-specific sequence, a substrate-specific sequence, and a partial catalytic core sequence disposed between the facilitator-specific sequence and the substrate-specific sequence; and
(f) a second component oligonucleotide comprising a facilitator-specific sequence, a substrate-specific sequence, and a partial catalytic core sequence disposed between the facilitator-specific sequence and the substrate-specific sequence;
wherein the first component oligonucleotide and the second component form a catalytically active nucleic acid enzyme when hybridized to the facilitator nucleic acid and the substrate nucleic acid, and further wherein the catalytically active nucleic acid enzyme cleaves the substrate nucleic acid at a cleavage site to form a 5' fragment and a 3' fragment of the substrate nucleic acid.

2. A composition comprising:
(a) a facilitator nucleic acid;
(b) a substrate nucleic acid comprising a first label;
(c) a first primer complementary to a portion of the substrate nucleic acid and comprising a second label;
(d) a first component oligonucleotide comprising a facilitator-specific sequence, a substrate-specific sequence, and a partial catalytic core sequence disposed between the facilitator-specific sequence and the substrate-specific sequence; and
(e) a second component oligonucleotide comprising a facilitator-specific sequence, a substrate-specific sequence, and a partial catalytic core sequence disposed between the facilitator-specific sequence and the substrate-specific sequence;
wherein the first component oligonucleotide and the second component form a catalytically active nucleic acid enzyme when hybridized to the facilitator nucleic acid and the substrate nucleic acid, and further wherein the catalytically active nucleic acid enzyme cleaves the substrate nucleic acid at a cleavage site to form a 5' fragment and a 3' fragment of the substrate nucleic acid.

3. The composition of claim 1 wherein the facilitator nucleic acid is an amplicon and further comprising a primer pair and a target nucleic acid, wherein the primer pair primes the synthesis of the amplicon from the target nucleic acid.

4. The composition of claim 2 wherein the facilitator nucleic acid is an amplicon, and further comprising target nucleic acid and a primer pair, the primer pair comprising a second primer and a third primer, wherein the primer pair primes the synthesis of the amplicon from the target nucleic acid and Tms of both the second and third primers are above a predetermined Tm threshold, and the Tm of the first primer is below the predetermined threshold.

5. The composition of claim 1 or 2, wherein the substrate nucleic acid comprises deoxyribonucleotides and ribonucleotides, or wherein the substrate nucleic acid comprises one or more non-natural nucleotides.

6. The composition of claim 1 or 2, wherein the substrate nucleic acid comprises a fluorophore and a quencher, wherein the fluorophore and the quencher are disposed on different sides of the cleavage site.

7. The composition of claim 1, wherein the substrate nucleic acid further comprises a tail region at its 5' end, wherein the tail region is complementary to the capture probe and is not complementary to the first or second component oligonucleotides and optionally, wherein the tail region comprises one or more non-natural nucleotides.

8. The composition of claim 7, wherein the Tm of the substrate nucleic acid hybridized to the first and second component oligonucleotides is greater than the Tm of the 5' fragment of the substrate nucleic acid hybridized to the capture probe, which is greater than the Tm of the 5' fragment of the substrate nucleic acid hybridized to the first and second component oligonucleotides.

9. The composition of claim 1 or 2, wherein the cleavage site of the substrate nucleic acid comprises one or two ribonucleotides.

10. The composition of claim 1 or 2, wherein the facilitator-specific sequence of the first component oligonucleotide comprises between about 10 to 20 nucleotides complementary to the facilitator nucleic acid, or wherein the facilitator-specific sequence of the second component oligonucleotide comprises between about 10 to 20 nucleotides complementary to the facilitator nucleic acid.

11. The composition of claim 1 or 2, wherein the substrate-specific sequence of the first component oligonucleotide comprises between about 6 to 24 nucleotides complementary to the substrate nucleic acid, or wherein the substrate-specific sequence of the second component oligonucleotide comprises between about 6 to 24 nucleotides complementary to the substrate nucleic acid.

12. The composition of claim 1 or 2, wherein the partial catalytic core sequence of the first component oligonucleotide comprises between about 2 to 12 nucleotides, or wherein the partial catalytic core sequence of the second component oligonucleotide comprises between about 2 to 12 nucleotides.

13. The composition of claim 1, wherein the capture probe comprises a first region that is complementary to the 5'-fragment of the substrate nucleic acid, a second region that provides a mismatch to the 3'-terminal nucleotide of the 5'-fragment of the substrate nucleic acid, and a third region that provides a template sequence for nucleic acid synthesis from the 3'-end of the 5'-fragment of the substrate nucleic acid.

14. The composition of claim 1, wherein the composition comprises at least two different facilitator nucleic acids; at least two different substrate nucleic acids; at least two different capture probes, at least two different first component oligonucleotides, and at least two different second component oligonucleotides, wherein the at least two different substrate nucleic acids are labeled with the same label but have nucleic acid sequences that differ from each other and are complementary to their respective complementary sequences in the at least two different capture probes, and wherein the at least two different capture probes can be distinguished by Tm when hybridized to a complementary sequence.

15. The composition of claim 3 or 4, wherein at least one primer of the primer pair comprises:
(a) a non-hybridizing region comprising natural nucleotides that are not complementary to the target nucleic acid;
(b) an anchor region 5' of the non-hybridizing region, wherein the anchor region is complementary to a first portion of the target nucleic acid; and
(c) a footer region 3' of the non-hybridizing region, wherein the footer region is complementary to a second portion of the target nucleic acid, and further wherein the Tm of the footer region hybridized to the second portion of the target nucleic acid is lower than the Tm of the anchor region hybridized to the first portion of the target nucleic acid; and
(d) a non-natural nucleotide region located adjacent the 5'-end of the footer region, within the footer region, or within the non-hybridizing region.

16. The composition of claim 15, wherein the at least one primer comprises two non-natural nucleotide regions, wherein one of the non-natural nucleotide regions is located adjacent the 5'-end of the footer region or within the footer region, and the other of the non-natural nucleotide regions is located within the non-hybridizing region, or wherein the facilitator-specific sequence of either the first or second component oligonucleotide comprises a sequence complementary to the non-hybridizing region of the at least one primer.

17. The composition of claim 15, wherein the non-natural nucleotides in the non-natural nucleotide region are independently selected from the group consisting of isoC and isoG.

18. The composition of claim 2, wherein the first label is a fluorophore and the second label is a quencher, or wherein the first label is a quencher and the second label is a fluorophore.
